(19) Europäisches Patentamt
European Patent Office
Office européen des brevets

(11) Publication number: **0 204 848**
**B1**

(12) # EUROPEAN PATENT SPECIFICATION

(45) Date of publication of patent specification: **23.11.89**

(51) Int. Cl.[4]: **C 07 D 213/61**

(21) Application number: **85106739.7**

(22) Date of filing: **31.05.85**

(54) **Preparation of symmetrical tetrachloropyridine from chlorinated beta - (trichloromethyl) pyridines employing a catalyst.**

(43) Date of publication of application:
**17.12.86 Bulletin 86/51**

(45) Publication of the grant of the patent:
**23.11.89 Bulletin 89/47**

(84) Designated Contracting States:
**BE CH DE FR GB IT LI NL**

(56) References cited:
**EP-A-0 005 064**
**US-A-3 538 100**

**HELVETICA CHIMICA, vol. 59, fasc. 1, no. 20-21, 1976, pages 190-211, Basel, CH; U. HORN et al.: "Halogenierte pyridine II. Reaktionen der 3-Halogenmethyl-pyridine"**

**The file contains technical information submitted after the application was filed and not included in this specification**

(73) Proprietor: **THE DOW CHEMICAL COMPANY**
**2030 Dow Center Abbott Road P.O. Box 1967**
**Midland Michigan 48640-1967 (US)**

(72) Inventor: **Humphreys, Paula Lee**
**176 Woodview Terrace Drive**
**San Ramon California 94583 (US)**
Inventor: **Dietsche, Thomas J.**
**30 Roancke Road**
**Berkeley California 94705 (US)**

(74) Representative: **Weickmann, Heinrich, Dipl.-Ing. et al**
**Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 204 848 B1**

Courier Press, Leamington Spa, England.

**Description**

The present invention relates to the preparation of symmetrical tetrachloropyridine, i.e., 2,3,5,6-tetrachloropyridine, hereinafter referred to as "sym-tet", from chlorinated β-(trichloromethyl)pyridines in a catalyzed reaction.

Sym-tet is a well known compound useful as a chemical intermediate in the preparation of a variety of pesticides. Previous methods for preparing sym-tet include those described in U.S. Patent Nos. 3,538,100; 3,186,994; 4,225,778 and 4,256,894. Sym-tet is produced according to U.S. Patent 3,186,994 by chlorinating, in the absence of a catalyst, a polychloro-α-(trichloromethyl)pyridine in the liquid or gas phase. According to U.S. Patent 4,256,894 (equivalent to EP—A—0,005,064), sym-tet is prepared by reacting a chloro substituted α-(trichloromethyl)pyridine in the liquid state with chlorine at temperatures of at least 160°C in the presence of a catalyst.

Subject of the present invention is a method of preparing 2,3,5,6-tetrachloropyridine according to claim 1. Preferred embodiments of this process are subject matter of claims 2 to 8.

In accordance with the present invention, sym-tet is prepared in good yield by contacting a chlorinated β-(trichloromethyl)pyridine with chlorine and from 0.1 to 1.0 mole % catalyst in a liquid phase reaction under conditions sufficient to form sym-tet. The reaction is conducted at a temperature of at least 160°C and optionally at a superatmospheric pressure. The present process may be carried out in a batch manner but is preferably conducted in a continuous, cyclical operation to produce sym-tet in a commercially viable yield.

The present invention offers advantages over known methods of preparing sym-tet which include: (1) less formation of the by-product pentachloropyridine and (2) an accelerated rate of reaction.

In conducting the process of the present invention, gaseous chlorine is passed into a liquid chlorinated β-(trichloromethyl)pyridine starting material at a temperature of at least 160°C in the presence of a catalyst. An amount of chlorine gas reactant is employed that is sufficient to fully chlorinate the pyridine reactant to sym-tet. Usually at least an equimolar amount of chlorine is employed while an excess of up to 10 molar proportions or more of chlorine per mole of pyridine starting material desirably being employed. The most suitable rate at which the chlorine gas is fed will vary with such factors as reaction temperature, pressure, reaction mixture volume, starting material, etc. and readily determinable to one skilled in the art.

The catalyst is added to the present reactants to accelerate the rate of reaction. The catalysts comprise the Lewis acid type catalysts selected from the halides of Fe, W, Mo, Ti and Sb, as described in I.S. Patent 4,256,894. The catalyst is employed in an amount ranging from 0.1 to 1 mole percent based on the amount of chlorinated β-(trichloromethyl)pyridine starting material. Preferably, a catalyst concentration of from 0.3 to 0.5 mole percent, most advantageously about 0.4 mole percent, is employed. Preferred catalysts are iron powder and $FeCl_3$. When iron powder is employed as the catalyst it reacts with $Cl_2$ in the reaction mixture to form $FeCl_2$ *in situ*.

While the pressure at which the present reaction is conducted is not critical, a superatmospheric pressure is preferably employed. Generally speaking, the higher the pressure, the faster the rate of formation of sym-tet. The present reaction is advantageously run at a pressure of from 204.746 to 6996.082 kPa (15 psig to 1,000 psig) and preferably from 273.694 to 2859.229 kPa (25 psig to 400 psig). A particularly preferred pressure to run the present process is about 1480.277 kPa (200 psig).

Chlorinated β-(trichloromethyl)pyridine employed as starting materials are all well known compounds and are represented by the following formula:

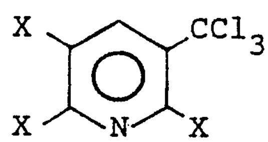

wherein each X independently represents H or Cl with the proviso that at least one X is always Cl. The chlorinated β-(trichloromethyl)pyridine starting materials may be supplied as pure compounds and reacted according to the present invention, or, as is usually the case, a mixture containing more than one chlorinated β-(trichloromethyl)pyridine compounds is reacted according to the present invention to form sym-tet. These mixtures of chlorinated β-(trichloromethyl)pyridines are obtained as by-products in the preparation of valuable β-(trichloromethyl)pyridines, such as, 2,3-dichloro-5-(trichloromethyl)pyridine and 2-chloro-5-(trichloromethyl)pyridine, which are chemical intermediates used in preparing herbicides.

In carrying out the present invention, the chlorinated β-(trichloromethyl)pyridine material in the liquid form, is added to the reaction vessel. An effective amount of a catalyst is added to the reaction vessel and then the chlorine flow is commenced, usually at a sufficient rate to pressurize the reaction vessel to about 204.746 kPa (15 psig) or more. The temperature of the reactants is then slowly increased to at least 160°C or more and the reaction maintained until sufficient amounts of sym-tet are obtained. Liquid samples from the reaction vessel and vent gases are periodically taken and analyzed employing known methods to monitor the course of the reaction. The reaction is terminated by stopping the heating of the reaction vessel and the flow of chlorine thereto and allowing the reaction vessel pressure to drop to atmospheric pressure. The desired sym-tet is then recovered employing known separatory or purification techniques such as distillation.

Alternatively the temperatures of the reactants may be raised to the desired level before the introduction of chlorine gas begins.

If the present reaction is allowed to run too long formation of pentachloropyridine, a by-product, will result, thus decreasing the yield of sym-tet. It is readily apparent to one skilled in the art that the reaction be run for an amount of time which maximizes the yield of sym-tet. The optimum reaction time will depend on a variety of factors, such as, for example, specific starting materials employed, pressure, temperature, amounts of rectants employed, and rate of the chlorine feed, to name a few. Each operation of the present invention is monitored as described above to determine the optimum reaction time for that particular operation.

In one embodiment of the present invention one or more chlorinated β-(trichloromethyl)pyridines are reacted with chlorine in the presence of a catalyst at atmospheric pressure and at a temperature of at least 160°C. For economical reasons, this reaction is preferably run at superatmospheric pressures usually from 204.746 to 1718.172 kPa (15 to 220 psig) or more, which increases the rate of the reaction. In a preferred embodiment, the reaction is conducted at temperatures of from 160°C to 220°C and pressures of 790.801 kPa (100 psig) to 1718.172 kPa (220 psig) or more. In an especially preferred embodiment, a reaction temperature of about 200°C and a reaction pressure of about 1480.277 kPa (200 psig) are employed whereby optimum yields of sym-tet can be obtained in a batch reaction.

In another preferred embodiment, a mixture of chlorinated β-(trichloromethyl)pyridines, containing as a major portion 2,6-dichloro-3-(trichloromethyl)pyridine and/or 2-chloro-5-(trichloromethyl)pyridine, is employed as the chlorinated β-(trichloromethyl)pyridine starting material and reacted with chlorine in the presence of a catalyst at a temperature of from 160°C to 200°C and at a pressure of from 790.801 to 1718.172 kPa (100 psig to 220 psig). The sym-tet prepared is then recovered by distillation. Similar results are obtained employing mixtures containing 2,3,6-trichloro-5-(trichloromethyl)pyridine and/or 2,3-dichloro-5-(trichloromethyl)pyridine,

In all embodiments fo the present invention, it is to be noted that the only contraint placed upon the superatmospheric pressures employed is one of economics and that pressures in excess of the preferred range may be employed.

The following examples illustrate the present invention. All percentages are in weight percent unless indicated otherwise.

Example 1

A mixture (778.6 grams) of chlorinated β-picolines containing 38.3 weight percent of 2,6-dichloro-3-(trichloromethyl)pyridine, 31.4 weight percent of 2-chloro-5-(trichloromethyl)pyridine, 17.9 percent of 2,3,6-trichloropyridine, 2.0 weight percent of 2,3,5,6-tetrachloropyridine, 2.0 weight percent of 2,3-dichloro-5-(trichloromethyl)pyridine, 0.4 weight percent of pentachloropyridine and 8.0 weight percent of other chlorinated β-picolines, together with 2.25 grams (0.4 mole %) of $FeCl_2$ catalyst, was chlorinated in the liquid phase in a Parr bomb at 200°C and 1480.277 kPa (200 psig) by bubbling chlorine into the mixture at the rate of about 20.9 grams/hour. After 71.75 hours the reaction mixture contained the following:

| | Wt.% |
|---|---|
| 2,3,5,6-tetrachloropyridine | 40.1 |
| 2,3,6-trichloropyridine | 38.1 |
| pentachloropyridine | 4.7 |
| other chlorinated pyridines | 14.4 |

The ratio of pentachloropyridine to 2,3,5,6-tetrachloropyridine was 0.12.

Example 2—13

Substantially the same procedures conducted in Example 1 were repeated employing different mixtures of chlorinated β-picolines. The exact conditions are listed in Table 1. The weight percent of the chlorinated β-picolines present in the starting mixture and product mixture is listed in Table 1.

**Table I**

| Run No. | Starting Material, g | Average Mole % $FeCl_3$ | Temp °C | Press. psig * | $Cl_2$ Flow g/hr | Run Time, Hours | Weight Percent | | | | | | | |
|---|---|---|---|---|---|---|---|---|---|---|---|---|---|---|
| | | | | | | | 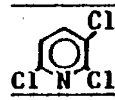 | 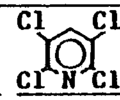 | 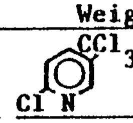 | 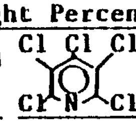 | 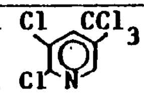 | 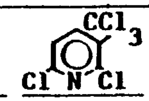 | 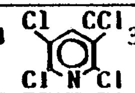 | Other |
| 2 | 442.0 | 1.0 | 200 | 200 | 16 | 0 | 13 | 2 | 32 | 0.4 | 2 | 26 | 1 | 24 |
| | | | | | | 48 | --- | 57 | 0.1 | 8 | 4 | 0.1 | 12 | 19 |
| 3 | 648.6 | 0.36 | 200 | 200 | 18 | 0 | --- | --- | 0.3 | 0.7 | 0.2 | 96.9 | 0.5 | 1.9 |
| | | | | | | 12 | 8.4 | 2.6 | 0.2 | 0.7 | 0.3 | 63.8 | 13.0 | 11.0 |
| | | | | | | 24 | 12.8 | 10.5 | 0.2 | 0.9 | 0.4 | 44.5 | 24.3 | 6.4 |
| 4 | 1115.8 | 0.28 | 200 | 200 | 25 | 0 | 14.7 | 1.7 | 33.0 | 0.4 | 1.7 | 26.3 | 0.9 | 21.3 |
| | | | | | | 24 | 17.3 | 5.4 | 7.6 | 1.2 | 13.2 | 29.9 | 7.9 | 17.5 |
| | | | | | | 48 | 14.9 | 19.1 | 1.4 | 2.8 | 11.1 | 16.2 | 18.7 | 15.8 |
| | | | | | | 78 | 8.4 | 38.0 | 0.2 | 5.3 | 7.4 | 5.1 | 20.9 | 14.5 |
| 5 | 2231 | 0.28 | 185 | 200 | 18 | 0 | 13.6 | 1.6 | 35.1 | 0.3 | 1.7 | 31.7 | 0.9 | 15.1 |
| | | | | | | 12 | 14.0 | 2.1 | 20.3 | 0.7 | 11.4 | 30.9 | 1.9 | 18.7 |
| | | | | | | 30 | 13.5 | 2.3 | 11.4 | 0.9 | 16.2 | 32.6 | 3.1 | 20.0 |
| | | | | | | 55 | 16.8 | 4.4 | 3.1 | 1.4 | 17.5 | 30.9 | 8.5 | 17.4 |
| | | | | | | 103 | 18.7 | 7.7 | 1.5 | 1.5 | 15.9 | 25.9 | 12.2 | 16.6 |
| | | | | | | 169 | 19.0 | 19.4 | 0.4 | 3.5 | 11.5 | 14.6 | 16.7 | 14.9 |

---indicates none detected

**Table I (Continued)**

| Run No. | Starting Material, g | Average Mole % $FeCl_3$ | Temp °C | Press. psig | $Cl_2$ Flow g/hr | Run Time, Hours | Weight Percent | | | | | | | |
|---|---|---|---|---|---|---|---|---|---|---|---|---|---|---|
| | | | | | | | 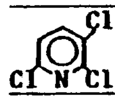 | 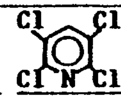 | 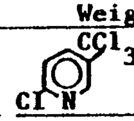 | 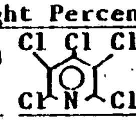 | 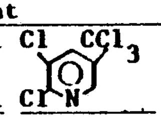 | 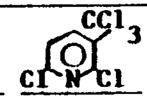 | 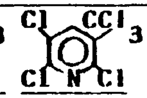 | Other |
| 6 | 2153.4 | 0.38 | 200 | 200 | 36 | 0 | 18.1 | 3.0 | 18.4 | 5.2 | 5.9 | 18.6 | 13.9 | 16.9 |
| | | | | | | 24 | 18.2 | 4.1 | 12.2 | 5.3 | 8.3 | 20.8 | 13.4 | 17.7 |
| | | | | | | 48 | 19.9 | 10.8 | 2.4 | 5.8 | 9.1 | 17.8 | 14.0 | 20.2 |
| | | | | | | 72 | 20.5 | 21.2 | 0.5 | 7.0 | 6.1 | 9.9 | 13.5 | 21.3 |
| | | | | | | 94 | 16.5 | 35.1 | --- | 8.4 | 3.1 | 4.4 | 11.3 | 21.2 |
| | | | | | | 130 | 6.4 | 57.4 | --- | 11.1 | 1.2 | 0.7 | 6.8 | 16.4 |
| 7 | 1124.8 | 0.56 | 200 | 200 | 29 | 0 | 17.7 | 8.9 | 28.7 | 1.2 | 2.5 | 22.5 | 7.4 | 11.1 |
| | | | | | | 24 | 23.5 | 15.2 | 5.8 | 2.1 | 10.8 | 26.0 | 11.3 | 5.3 |
| | | | | | | 42 | 27.9 | 24.8 | 1.2 | 3.1 | 7.9 | 16.9 | 12.6 | 5.6 |
| | | | | | | 69 | 22.5 | 41.4 | --- | 5.2 | 5.1 | 8.2 | 13.3 | 4.3 |
| | | | | | | 92 | 17.9 | 54.6 | --- | 6.5 | 3.1 | 3.5 | 9.9 | 4.5 |
| 8 | 2302.8 | 0.16 | 200 | 200 | 20 | 0 | 26.6 | 6.0 | 5.0 | 0.9 | 10.1 | 34.7 | 7.9 | 8.8 |
| | | | | | | 28 | 30.8 | 19.8 | 0.5 | 2.6 | 6.4 | 17.4 | 12.2 | 10.3 |
| | | | | | | 52 | 27.1 | 33.0 | --- | 3.9 | 3.9 | 8.3 | 11.0 | 12.8 |
| | | | | | | 76 | 20.3 | 49.5 | --- | 5.6 | 2.2 | 3.4 | 8.6 | 10.4 |

---indicates none detected

**Table I (Continued)**

| Run No. | Starting Material, g | Average Mole % $FeCl_3$ | Temp °C | Press psig | $Cl_2$ Flow g/hr | Run Time, Hours | Weight Percent | | | | | | | |
|---|---|---|---|---|---|---|---|---|---|---|---|---|---|---|
| | | | | | | | 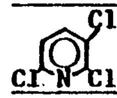 | 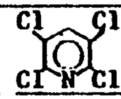 | 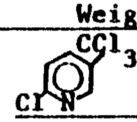 | 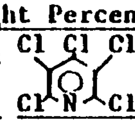 | 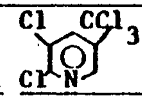 | 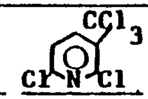 | 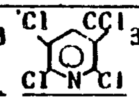 | Other |
| 9 | 647.3 | 0.36 | 200 | 200 | 105 | 0 | --- | --- | --- | 0.4 | 92.4 | --- | --- | 7.2 |
| | | | | | | 24 | --- | 4.4 | --- | 0.5 | 68.4 | 0.2 | 19.5 | 7.0 |
| | | | | | | 60 | --- | 29.7 | --- | 0.7 | 32.0 | --- | 34.5 | 3.1 |
| | | | | | | 88 | --- | 54.1 | --- | 1.1 | 17.2 | --- | 24.1 | 3.5 |
| 10 | 436.3 | 0.21 | 200 | 50 | 12 | 0 | --- | 0.1 | --- | --- | 96.1 | --- | 0.2 | 3.6 |
| | | | | | | 20 | --- | 8.7 | --- | 0.4 | 55.9 | --- | 22.5 | 12.5 |
| | | | | | | 33 | --- | 15.7 | --- | 0.6 | 43.4 | --- | 31.4 | 8.9 |
| | | | | | | 42 | --- | 20.7 | --- | 0.6 | 33.9 | --- | 29.7 | 15.1 |
| 11 | 549.1 | 0.15 | 200 | 200 | 21 | 0 | --- | --- | --- | --- | --- | --- | 84.9 | 15.1 |
| | | | | | | 8 | --- | 22 | --- | 0.6 | 0.2 | --- | 58.2 | 13.2 |
| | | | | | | 24 | --- | 52.0 | --- | 5.0 | --- | --- | 22.1 | 20.9 |
| 12 | 1194 | 0.31 | 200 | 200 | 7 | 0 | 17.3 | 1.7 | 30.3 | --- | 2.3 | 35.5 | 1.6 | 11.3 |
| | | | | | | 25 | 20.2 | 3.4 | 6.9 | 0.5 | 10.8 | 39.5 | 6.1 | 12.6 |
| | | | | | | 51 | 24.2 | 10.5 | 1.9 | 1.3 | 9.9 | 28.1 | 12.7 | 11.4 |
| | | | | | | 72 | 23.0 | 20.0 | 0.6 | 2.1 | 7.2 | 15.8 | 14.3 | 17.0 |
| | | | | | | 92 | 19.4 | 31.6 | 0.2 | 3.1 | 5.6 | 8.6 | 14.9 | 16.6 |

---indicates none detected

Table I (Continued)

| Run No. | Starting Material, g | Average Mole % $FeCl_3$ | Temp °C | Press. psig | $Cl_2$ Flow g/hr | Run Time, Hours | Weight Percent 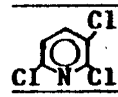 | 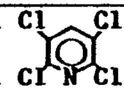 | 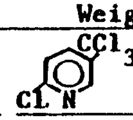 | 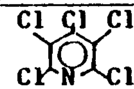 | 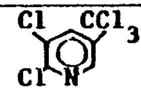 | 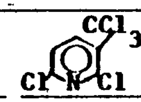 | 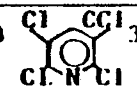 | Other |
|---|---|---|---|---|---|---|---|---|---|---|---|---|---|---|
| 13 | 1197.7 | 0.46 | 200 | 200 | 17 | 0 | 13.6 | 1.7 | 31.7 | 0.4 | 1.8 | 26.4 | 0.9 | 21.5 |
| | | | | | | 26 | 14.4 | 2.2 | 18.7 | 0.8 | 10.5 | 32.7 | 3.2 | 17.5 |
| | | | | | | 48 | 17.6 | 6.1 | 5.3 | 1.2 | 14.2 | 27.0 | 9.5 | 19.1 |
| | | | | | | 81 | 16.8 | 17.4 | 1.0 | 2.1 | 11.0 | 14.6 | 16.4 | 20.7 |

---indicates none detected
* 200 psig = 1480.277 kPa
50 psig = 546.063 kPa

Intermediates formed during the present reaction, such as, for example 2,3,6-trichloro-5-(trichloromethyl)pyridine and 2,3,6-trichloropyridine are conveniently recycled for more complete conversion to sym-tet employing techniques well known in the art.

In other embodiments of the present invention the reaction is conducted on a continuous recycle basis to prepare sym-tet. Such a process comprises the continuous chlorination of chlorinated β-(trichloromethyl)pyridines at temperatures of at least 160°C and at pressures of from atmospheric to 220 psig (1718.172 kPa) or more. Such a continuous process is conducted employing well known apparatus and procedures such as those described in U.S. Patent 4,256,894.

## Claims

1. A method of preparing 2,3,5,6-tetrachloropyridine which comprises contacting a compound or a mixture of more than one compound correponding to the formula

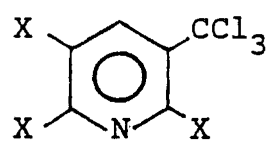

wherein each X independently represents H or Cl with the proviso that at least one X is always Cl, with chlorine in a liquid phase reaction in the presence of from 0.1 to 1.0 mole percent of a Lewis acid type catalyst selected from the halides of Fe, W, Mo, titanic chloride and antimony pentachloride, at a temperature of at least 160°C.

2. The method of Claim 1 wherein the chlorinated β-(trichloromethyl)pyridine is 2,6-dichloro-3-(trichloromethyl)pyridine, 2-chloro-5-(trichloromethyl)pyridine, 2,3,6-trichloro-5-(trichloromethyl)pyridine or 2,3-dichloro-5-(trichloromethyl)pyridine.

3. The method of Claim 1 wherein the pressure is from 790.801 kPa to 1718.172 kPa (100 to 220 psig).

4. The method of Claim 1 wherein the temperature is from 160°C to 200°C.

5. The method of Claim 4 wherein the catalyst is present in an amount of from 0.3 to 0.5 mol percent based on the amount of chlorinated β-(trichloromethyl)pyridine present in the reaction mixture.

6. The method of Claim 5 wherein the catalyst is a ferric halide.

7. The method of Claim 6 wheren the ferric halide is $FeCl_2$.

8. The method of Claim 7 wherein the ferric chloride is formed *in situ* by adding iron powder to the chlorinated β-(trichloromethyl)pyridine starting material.

## Patentansprüche

1. Verfahren zur Herstellung von 2,3,5,6-Tetrachlorpyridin, worin eine Verbindung oder ein Gemisch aus mehr als einer Verbindung entsprechend folgender Formel

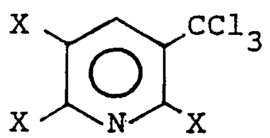

wobei jedes X voneinander unabhängig E oder Cl darstellen soll, vorbehaltlich, daß immer mindestens ein X für Cl steht, in einer Flüssigphasen-Reaktion mit Chlor in Kontakt gebrecht wird in Gegenwart won 0,1 bis 1,0 Mol-% eines Lewis-Säureartigen Katalysators, der aus den Halogeniden von Fe, W and Mo, Titanchlorid und Antimonpentachlorid ausgewählt wird, bei einer Temperatur von mindestens 160°C.

2. Verfahren nach Anspruch 1, worin das chlorierte ß-Trichlormethylpyridin 2,4-Dichlor-3-(trichlormethyl)-pyridin, 2-Chlor-3-(trichlormethyl)-pyridin, 2,3,6-Trichlor-5-(trichlormethyl)-pyridin oder 2,3-Dichlor-5-(trichlormethyl)-pyridin ist.

3. Verfahren nach Anspruch 1, worin der Druck von 790, 801 kPa bis 1718, 172 kPa (100 bis 220 psig) ist.

4. Verfahren nach Anspruch 1, worin die Temperatur von 160°C bis 200°C ist.

5. Verfahren nach Anspruch 4, worin der Katalysator in einer Menge von 0,3 bis 0,5 Mol-%, bezogen auf die Menge von chloriertem ß-Trichlormethylpyridin, in der Reaktionsmischung anwesend ist.

6. Verfahren nach Anspruch 5, worin der Katalysator ein Eisenhalogenid ist.

7. Verfahren nach Anspruch 6, worin das Eisenhalogenid $FeCl_3$ ist.

8. Verfahren nach Anspruch 7, worin das Eisenchlorid *in situ* durch die Zugabe von Eisenpulver zum chlorierten ß-Trichlormethylpyridin-Ausgangsmaterial gebildet wird.

**Revendications**

1. Procédé de préparation de 2,3,5,6-tétrachloropyridine, qui consiste à faire réagir un composé ou un mélange de plus d'un composé correspondant à la formule

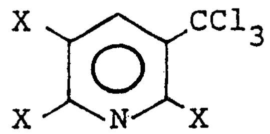

dans laquelle chaque X indépendamment représente H ou Cl, avec la condition qu'au moins un X soit toujours Cl, avec du chlore, dans une réaction en phase liquide en présence de 0,1 à 1,0 mole pour cent d'un catalyseur de type acide de Lewis choisi parmi les halogénures de Fe, W, Mo, chlorure titanique est le pentachlorure d'antimoine, à une température d'au moins 160°C.

2. Procédé selon la revendication 1, dans lequel la β-(trichlorométhyl)pyridine chlorée est la 2,6-dichloro-3-(trichlorométhyl)pyridine, la 2-chloro-5-(trichlorométhyl)pyridine, la 2,3,6-trichloro-5-(trichlorométhyl)pyridine ou la 2,3-dichloro-5-(trichlorométhyl)pyridine.

3. Procédé selon la revendication 1, dans lequel la pression est de l'ordre de 790,801 kPa à 1.718,172 kPa (100 à 220 psig).

4. Procédé selon la revendication 1, dans lequel la température est de l'ordre de 160°C à 200°V.

5. Procédé selon la revendication 4, dans lequel le catalyseur est présent en une proportion entre 0,3 et 0,5 mole pour cent par rapport à la quantité de β-(trichlorométhyl)pyridine chlorée présente dans le mélange réactionnel.

6. Procédé selon la revendication 5, dans lequel le catalyseur est un halogénure ferrique.

7. Procédé selon la revendication 6, dans lequel l'halogénure ferrique est $FeCl_3$.

8. Procédé selon la revendication 7, dans lequel le chlorure ferrique est formé in situ par addition de poudre de fer à la β-(trichlorométhyl)pyridine chlorée de départ.